(19) Europäisches Patentamt
European Patent Office
Office européen des brevets

(11) **EP 4 394 687 A1**

(12) **EUROPEAN PATENT APPLICATION**
published in accordance with Art. 153(4) EPC

(43) Date of publication:
**03.07.2024   Bulletin 2024/27**

(21) Application number: **22860903.8**

(22) Date of filing: **16.05.2022**

(51) International Patent Classification (IPC):
***G06Q 50/10*** *(2012.01)*        ***A61B 10/00*** *(2006.01)*

(52) Cooperative Patent Classification (CPC):
**A61B 10/00; G06Q 50/10**

(86) International application number:
**PCT/JP2022/020381**

(87) International publication number:
**WO 2023/026606 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **27.08.2021   JP 2021138800**

(71) Applicant: **Shimadzu Corporation
Nakagyo-ku,
Kyoto-shi,
Kyoto 604-8511 (JP)**

(72) Inventors:
• **OSAKI, Kaori
Kyoto-shi, Kyoto 604-8511 (JP)**
• **INOUE, Yoshihiro
Kyoto-shi, Kyoto 604-8511 (JP)**

(74) Representative: **Müller-Boré & Partner
Patentanwälte PartG mbB
Friedenheimer Brücke 21
80639 München (DE)**

(54) **BRAIN WORKLOAD INDEX CALCULATION SYSTEM AND BRAIN WORKLOAD INDEX CALCULATION METHOD**

(57)     A brain workload index calculation system (100) includes a brain activity measurement device (1) configured to measure brain activity of a subject (P), a data processor (21) configured or programmed to calculate a brain workload index based on a degree of change in measurement data of the brain activity of the subject during execution of a plurality of tasks with different difficulty levels with respect to work performance of the subject for each of the plurality of tasks with different difficulty levels, the measurement data being acquired from the brain activity measurement device (1), and a display (24) configured to display the brain workload index calculated by the data processor (21).

*FIG.2*

EP 4 394 687 A1

# Description

## Technical Field

The present invention relates to a brain workload index calculation system and a brain workload index calculation method, and more particularly, it relates to a brain workload index calculation system including a brain activity measurement device, a data processor, and a display, and a brain workload index calculation method.

## Background Art

Conventionally, an optical measurement device including a brain activity measurement device, a data processor, and a display is known. Such an optical measurement device is disclosed in Japanese Patent Laid-Open No. 2015-229095, for example.

Japanese Patent Laid-Open No. 2015-229095 discloses an optical measurement device that performs optical measurement (brain function measurement) using near-infrared spectroscopy (NIRS). This optical measurement device includes an optical measurement unit (brain activity measurement device) and a control unit (data processor) including a display. Light transmitting probes and light receiving probes of the optical measurement device are placed at predetermined positions on the head surface of a subject by being attached to a probe fixing holder attached to the head of the subject. The optical measurement device then emits measurement light in a near-infrared light wavelength range from the light transmitting probes and acquires the intensity of the measurement light (the amount of received light) by allowing the measurement light reflected in the head of the subject to enter the light receiving probes and detecting the measurement light reflected in the head.

The optical measurement device can acquire a change in a hemoglobin amount (oxygenated hemoglobin, deoxygenated hemoglobin, and total hemoglobin) associated with brain activity based on the acquired intensity of the measurement light.

## Prior Art

### Patent Document

Patent Document 1: Japanese Patent Laid-Open No. 2015-229095

## Summary of the Invention

### Problems to be Solved by the Invention

Although not disclosed in Japanese Patent Laid-Open No. 2015-229095, it is conceivable to use data indicating the state of the brain during work, for example, in skill evaluation of workers. However, in the skill evaluation of workers, there has been no index showing brain workload to objectively judge or evaluate the state of the brain during work, for example. Therefore, there is a desire for an index that shows brain workload that can be used as objective data that indicates the state of the brain.

The present invention is intended to solve at least one of the above problems. The present invention aims to provide a brain workload index calculation system and a brain workload index calculation method each capable of presenting an index showing brain workload that can be used as objective data indicating the state of the brain when a worker performs work.

### Means for Solving the Problems

A brain workload index calculation system according to a first aspect of the present invention includes a brain activity measurement device configured to measure brain activity of a subject, a data processor configured or programmed to calculate a brain workload index based on a degree of change in measurement data of the brain activity of the subject during execution of a plurality of tasks with different difficulty levels with respect to work performance of the subject for each of the plurality of tasks with different difficulty levels, the measurement data being acquired from the brain activity measurement device, and a display configured to display the brain workload index calculated by the data processor. The "work performance" is a score representing an evaluation of a result for a task performed by the subject during brain activity measurement.

A brain workload index calculation method according to a second aspect of the present invention includes acquiring measurement data of brain activity of a subject during execution of a plurality of tasks with different difficulty levels, acquiring work performance of the subject for each of the plurality of tasks with different difficulty levels, calculating a brain workload index based on a degree of change in acquired measurement data of the brain activity of the subject with respect to acquired work performance of the subject, and displaying a calculated brain workload index.

### Effect of the Invention

In the brain workload index calculation system according to the first aspect of the present invention, as described above, the data processor calculates the brain workload index based on the degree of change in the measurement data of the brain activity of the subject during execution of the plurality of tasks with different difficulty levels with respect to the work performance of the subject for each of the plurality of tasks with different difficulty levels, acquired from the brain activity measurement device. The degree of change in the measurement data of the brain activity of the subject with respect to the work performance of the subject is acquired as the brain workload index such that it can be evaluated that the larger degree of change acquired is closer to changes in the brain activity of young people. Therefore, it is possible to present an index showing the brain workload that can be used as objective data indicating the state of the brain when a worker performs work.

As described above, the brain workload index calculation method according to the second aspect of the present invention includes calculating the brain workload index based on the degree of change in the measurement data of the brain activity of the subject during execution of the plurality of tasks with different difficulty levels with respect to the work performance of the subject for each of the plurality of tasks with different difficulty levels. Similarly to the first aspect described above, the degree of change in the measurement data of the brain activity of the subject with respect to the work performance of the subject is acquired as the brain workload index such that it can be evaluated that the larger degree of change acquired is closer to changes in the brain activity of young people. Therefore, it is possible to present an index showing the brain workload that can be used as objective data indicating the state of the brain when a worker performs work.

Brief Description of the Drawings

FIG. 1 is a schematic view showing the overall configuration of a brain workload index calculation system according to one embodiment of the present invention.

FIG. 2 is a block diagram of a brain workload index calculation system according to one embodiment of the present invention.

FIG. 3 is a schematic view showing an example of a screen displayed on a display according to one embodiment of the present invention.

FIG. 4 is a graph showing a young model and an elderly model for evaluating a difference in brain activity amount with respect to a difference in work performance.

FIG. 5 is a schematic view showing a measurement design that includes task execution periods and rest periods according to one embodiment of the present invention.

FIG. 6 is a graph for illustrating a plateau region.

FIG. 7 is a graph showing a first example of a change to a young model after training in evaluation of suitability of training.

FIG. 8 is a graph showing a second example of a change to a young model after training in evaluation of suitability of training.

FIG. 9 is a graph showing a third example of a change to a young model after training in evaluation of suitability of training.

FIG. 10 is a graph showing a first example of a change to an elderly model after training in evaluation of suitability of training.

FIG. 11 is a graph showing a second example of a change to an elderly model after training in evaluation of suitability of training.

FIG. 12 is a graph showing a third example of a change to an elderly model after training in evaluation of suitability of training.

FIG. 13 is a flowchart for illustrating a brain workload index calculation process by a controller.

FIG. 14 is a graph showing a first example of models for a product A and a product B in product evaluation.

FIG. 15 is a graph showing a second example of models for a product A and a product B in product evaluation.

FIG. 16 is a graph showing a third example of models for a product A and a product B in product evaluation.

Modes for Carrying Out the Invention

Embodiments embodying the present invention are hereinafter described on the basis of the drawings.

Configuration of Brain Workload Index Calculation System

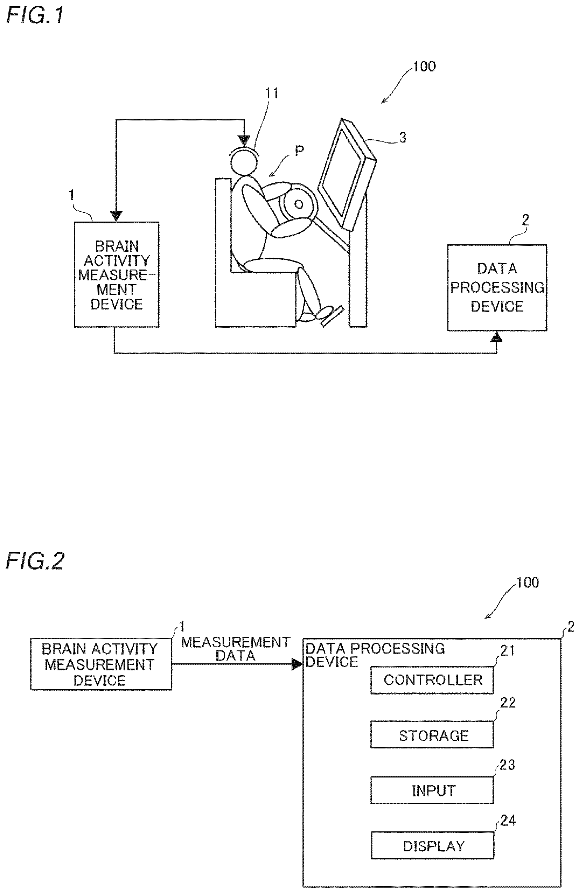

The overall configuration of a brain workload index calculation system 100 according to one embodiment of the present invention is now described with reference to FIGS. 1 to 9.

As shown in FIG. 1, the brain workload index calculation system 100 according to this embodiment includes a brain activity measurement device 1 and a data processing device 2.

The brain activity measurement device 1 is a device (optical measurement device) that optically measures the brain activity of a subject P using near-infrared spectroscopy (NIRS) and generates time-series measurement result data. Specifically, the brain activity measurement device 1 is a NIRS device. The brain activity measurement device 1 emits measurement light in a near-infrared light wavelength range from light transmitting probes (not shown) placed on the head surface of the subject P. Then, the brain activity measurement device 1 acquires the intensity of the measurement light (the amount of received light) by allowing the measurement light reflected in the head to enter light receiving probes (not shown) placed on the head surface and detecting the measurement light reflected in the head. A plurality of light transmitting probes and a plurality of light receiving probes are provided, and are attached to a holder 11 for fixing each probe at a predetermined position on the head surface. The brain activity measurement device 1 measures the amount of change in oxygenated hemoglobin, the amount of change in deoxygenated hemoglobin, and the amount of change in total hemoglobin based on the intensity of the measurement light (the amount of received light) at a plurality of wavelengths (three wavelengths of 780 nm, 805 nm, and 830 nm, for example) and the light absorption characteristics of hemoglobin. Thus, the brain activity measurement device 1 measures a change in the blood state of the brain associated with brain activity as a change in a hemoglobin amount.

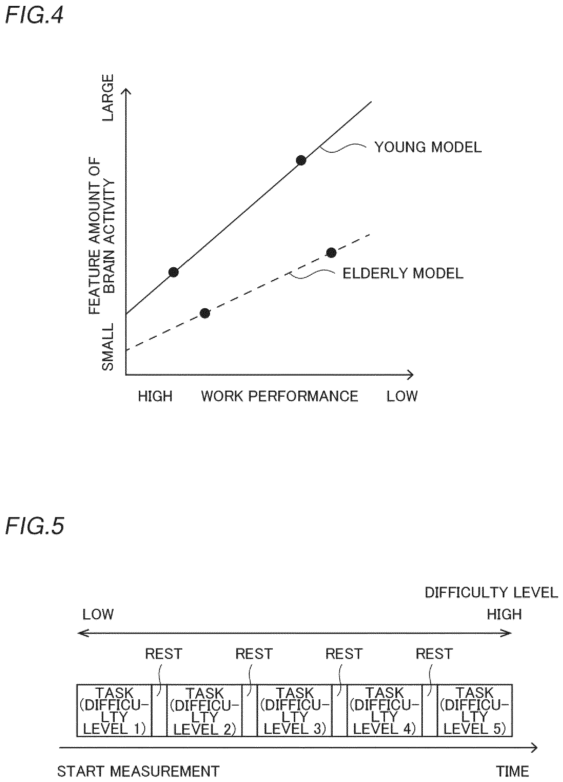

Brain activity measurement is performed while a task execution period in which each of a plurality of tasks with different difficulty levels is performed for each difficulty level and a rest period in which no task is executed are alternately repeated (see FIG. 5). In the brain activity measurement, a rest period may or may not be provided. Regions of the brain related to memory, cognition, motivation, judgment, etc. are set as measurement sites for acquiring measurement data of the brain activity of the subject P. For example, the prefrontal cortex of the brain is set as the measurement site. Specifically, the dorsolateral prefrontal cortex of the brain is set as the measurement site.

The brain activity measurement device 1 is communicably connected to the data processing device 2 and transmits the obtained measurement data to the data processing device 2.

The device configuration of the data processing device 2 is now described.

As shown in FIG. 2, the data processing device 2 is a personal computer (PC) that includes a controller 21 including a CPU, a memory, etc., a storage 22 such as a hard disk drive or a flash memory, an input 23 such as a keyboard, and a display 24. When the controller 21 (CPU) executes a program stored in the storage 22, the PC functions as the data processing device 2 of the brain workload index calculation system. The controller 21 acquires the measurement data through communication with the brain activity measurement device 1. Furthermore, the controller 21 acquires file data for data processing into which work performance has been input through the input 23. The work performance is a score representing an evaluation of a result for a task performed by the subject P during brain activity measurement. Specifically, the work performance is scores representing evaluations of results for the plurality of tasks with different difficulty levels performed by the subject P during brain activity measurement. The subject P is caused to perform the plurality of tasks set at different difficulty levels, and is also caused to perform a plurality of tasks at each difficulty level. Therefore, more specifically, the work performance is the average value of the work performance that is results for the plurality of tasks at each of a plurality of difficulty levels. The controller 21 is an example of a "data processor" in the present invention.

In this embodiment, the controller 21 calculates a brain workload index based on the degree of change in the measurement data of the brain activity of the subject P during execution of the plurality of tasks with different difficulty levels acquired from the brain activity measurement device 1 with respect to the work performance of the subject P for each of the plurality of tasks with different difficulty levels. The controller 21 calculates, as the brain workload index, the amount of change in brain activity in the measurement data with respect to the amount of change in order of work performance for each of the plurality of tasks with different difficulty levels. The amount of change in brain activity refers to the amount of change in oxygenated hemoglobin, the amount of change in deoxygenated hemoglobin, and the amount of change in total hemoglobin, for example. The brain workload index is described below in detail.

The input 23 receives an input by a user regarding a numerical value related to work performance. The work performance is input by the user into the file data for data processing according to the type of behavioral data and the subject P. The file data is data in Excel format, CSV format, or text format, for example.

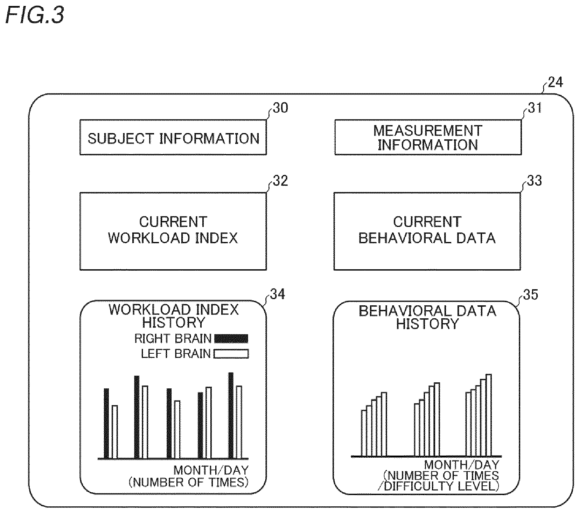

The display 24 displays various information including the brain workload index output from the controller 21. FIG. 3 is a schematic view showing an example of a screen displayed on the display 24. Examples of the information displayed on the display 24 include subject information 30, measurement information 31, a current brain workload index 32, current behavioral data 33, a workload index history 34, and a behavioral data history 35.

In this embodiment, calculation of the brain workload index for the same subject P is performed a plurality of times. The current brain workload index 32 refers to the amount of change in brain activity in the measurement data with respect to the amount of change in work performance for each of the plurality of tasks with different difficulty levels acquired by performing all of the plurality of tasks with different difficulty levels. Furthermore, the workload index history 34 is obtained by arranging, in chronological order, previously calculated brain workload indexes.

The current behavioral data 33 refers to the average value of the work performance that is results for the plurality of tasks at each difficulty level in the plurality of tasks with different difficulty levels, and the work performance is displayed according to the number of difficulty levels. Furthermore, the behavioral data history 35 is obtained by arranging, in chronological order, work performance according to the number of previously acquired difficulty levels.

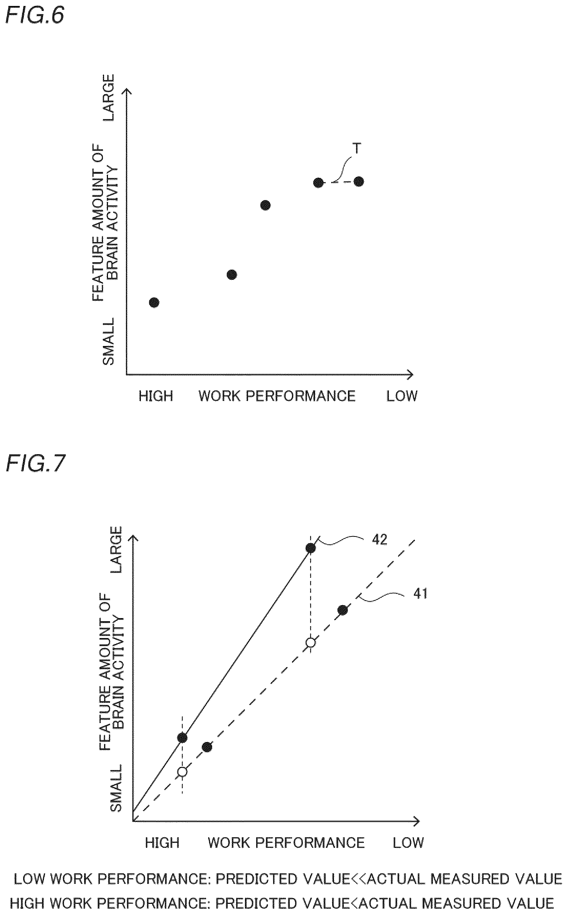

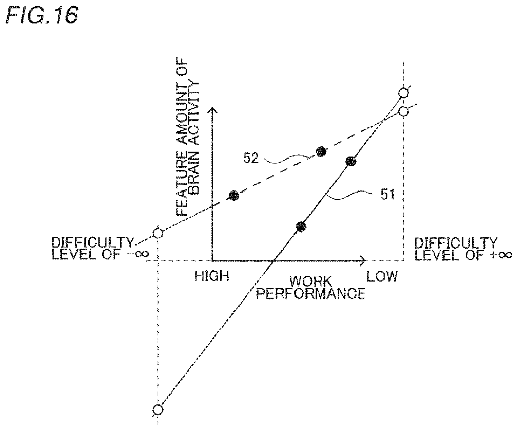

The information displayed on the display 24 includes graphs representing approximate straight lines (see FIGS. 7 to 12 and 14 to 16) described below, the presence or absence of a plateau region T (see FIG. 6) as a stagnant region described below, etc. As long as at least the current brain workload index 32 is displayed on the display 24, any of the remaining information may not be displayed on the display 24.

As the current brain workload index 32, a value (numerical value) of the amount of change in brain activity in the measurement data with respect to the amount of change in work performance for each of the plurality of tasks with different difficulty levels is displayed on the display 24. As the current brain workload index 32, a preset mark or color based on the above value may be displayed on the display 24. Examples of the mark include "0", "Δ", and "x" depending on the value. As for the color, for example, when values (numerical values) of the amounts of change in brain activity with respect to the amounts of change in work performance before and after training, which are described later, are compared, and the value of the amount of change in brain activity with respect to the amount of change in work performance after the training is larger, the above value is displayed in a dark color. When the value of the amount of change in brain activity with respect to the amount of change in work performance after the training is smaller, the above value is displayed in a light color.

The name, gender, age, etc. of the subject P are displayed on the display 24 as the subject information 30. As the measurement information 31, the date and time of brain activity measurement by the brain activity measurement device 1, the measurement site, etc. are displayed on the display 24. As the current behavioral data 33, the work performance of the subject P for each of the plurality of tasks with different difficulty levels is displayed on the display 24. As the workload index history 34, the brain workload index calculated in the past for the same subject P is displayed on the display 24. As the behavioral data history 35, the work performance for each of the plurality of tasks with different difficulty levels acquired in the past in the same subject P is displayed on the display 24. The workload index history 34 and the behavioral data history 35 may be displayed not as bar graphs but as line graphs.

Brain Workload Index

The brain workload index is now described.

The brain workload index is based on a model for evaluating differences in brain activity amount with respect to differences in work performance. FIG. 4 is a graph in which the vertical axis (explained variable) represents a feature amount (brain activity amount) in brain activity, and the horizontal axis (explanatory variable) represents work performance. A large feature amount in brain activity indicates that the brain activity amount is large, and a small feature amount in brain activity indicates that the brain activity amount is small.

FIG. 4 shows a young model (solid line), which is an example of a model for young people, and an elderly model (broken line), which is an example of a model for elderly people, as models for evaluating differences in brain activity with respect to differences in work performance. When the young model is compared with the elderly model, differences in brain activity with respect to differences in work performance in the young model are greater than those in the elderly model. That is, the young model has a larger slope, which is the amount of change in brain activity with respect to the amount of change in work performance, than the elderly model. Furthermore, in the young model, the brain has a stronger response (larger brain activity amount) when the work performance is low (high difficulty level) than in the elderly model. As the brain workload index, the young model is preferable to the elderly model.

In the graph representing the young model and the elderly model, the work performance is set as the horizontal axis. For example, even when tasks are set at the same difficulty level, some subjects P may find the task difficult, while others may find the task easy. Furthermore, even when the subject P becomes accustomed to the task after performing the task with the same difficulty level a plurality of times, the task will be treated as "the same difficulty level", and the influence of habituation will not be taken into consideration. In order to reduce or prevent the influence of differences in how the subjects P perceive difficulty and their habituation, and to obtain a more objective index that does not depend on "difficulty", a model with work performance as the horizontal axis is used.

Outline of Training, Task, Task Difficulty Level, and Work Performance

The training includes driving training using a drive simulator 3 (see FIG. 1) at a driving license school, for example. Furthermore, the training includes flight training for pilots using a flight simulator (not shown), for example. The training is not limited to the driving training and flight training described above.

The measurement data of the brain activity of the subject P measured by the brain activity measurement device 1 is measured during execution of a plurality of tasks with different difficulty levels. When the training is driving training using the drive simulator 3 (see FIG. 1), the task is to perform a simulated driving using the same drive simulator 3 as the training. When the training is flight training using a flight simulator (not shown), the task is to perform a simulated flight using the same flight simulator as the training. The task may be the same in content as the training or may be different in content from the training.

When the brain activity is measured by the brain activity measurement device 1, the subject P is caused to perform a plurality of tasks with different difficulty levels. As shown in FIG. 5, the tasks are performed in order from a task with the lowest difficulty level to a task with the highest difficulty level. Tasks with at least two different difficulty levels are performed. It is better to have a large number of tasks with different difficulty levels, but there is no particular limitation as long as there are two or more tasks. In this embodiment, tasks with five different difficulty levels are performed. Furthermore, the task at each difficulty level is performed a plurality of times for each difficulty level. In this embodiment, the task at each difficulty level is performed five times for each difficulty level. A difference in difficulty level between a plurality of tasks with different difficulty levels is preferably larger. The rest period is set between a plurality of tasks with different difficulty levels. The rest period may or may not be set.

As the work performance at each difficulty level, the average value of the work performance in the task performed a plurality of times for each difficulty level is used. As the brain activity amount in the task for each difficulty level, the average value or integral value of oxygenated hemoglobin, deoxygenated hemoglobin, and total hemoglobin in the task performed a plurality of times for each difficulty level is used, for example.

For example, when the task is to perform a simulated driving using the drive simulator 3 (see FIG. 1), the difficulty level is set based on the difficulty level of a driving course. For example, the difficulty level is set depending on the number of events that occur during the simulated driving. Setting different difficulty levels for tasks is not limited to the above examples.

The work performance is a score representing the evaluation of the result for each of the plurality of tasks with different difficulty levels. For example, when the task is to perform a simulated drive using the drive simulator 3 (see FIG. 1), the work performance is obtained by converting various behavioral data, such as the time traveled at a constant speed and the time traveled from one section to another, into scores based on predetermined criteria.

Brain Workload Index Calculation Method

A brain workload index calculation method is now described.

The brain workload index is calculated based on a regression model between brain activity and work performance in graphs (see FIGS. 7 to 12) in which the vertical axis (explained variable) represents a feature amount in brain activity (brain activity amount) and the horizontal axis (explanatory variable) represents work performance, for example. An example of the brain workload index includes the amount of change in brain activity in the measurement data with respect to the amount of change in order of work performance for each of the plurality of tasks with different difficulty levels. The horizontal axis in the graph is set such that a distance from the origin increases, the work performance decreases. The vertical axis in the graph is set such that the distance from the origin increases, the amount of change in brain activity increases.

The controller 21 acquires the measurement data of the brain activity of the subject P from the brain activity measurement device 1. The controller 21 acquires the amount of change in brain activity as the measurement data of the brain activity. Specifically, the controller 21 acquires the amount of change in oxygenated hemoglobin, the amount of change in deoxygenated hemoglobin, and the amount of change in total hemoglobin as the measurement data of the brain activity. The controller 21 calculates a feature amount in a certain section of the measurement data for each of the plurality of tasks with different difficulty degrees. The feature amount is the average value or integral value of the amount of change in oxygenated hemoglobin, the average value or integral value of the amount of change in deoxygenated hemoglobin, and the average value or integral value of the amount of change in total hemoglobin, as the measurement data of the brain activity in the certain section of the measurement data described above.

The controller 21 acquires the work performance obtained by converting evaluations of the results for the plurality of tasks with different difficulty levels into scores, which is input through the input 23.

The controller 21 calculates an approximate straight line for plot points plotted on a graph as the brain activity amount with respect to the work performance for each of the plurality of tasks with different difficulty levels. The approximate straight line showing the relationship between work performance X and a brain activity amount (feature amount) Y is expressed by the following equation (1).

$$Y = aX + b \ ... \ (1)$$

The controller 21 acquires the slope a of the calculated approximate straight line as the brain workload index. Furthermore, the controller 21 acquires the intercept b of the calculated approximate straight line as a secondary brain workload index.

The controller 21 calculates a determination coefficient $R^2$ indicating the accuracy of the approximate straight line expressed by the above equation (1). The determination coefficient $R^2$ is calculated by the square of a correlation coefficient R expressed by the following equation (2). The controller 21 acquires the calculated determination coefficient $R^2$ as the secondary brain workload index.

$$R = \frac{\sum(x_i - \bar{x})(y_i - \bar{y})}{\sqrt{\sum(x_i - \bar{x})^2 \sum(y_i - \bar{y})^2}} \quad \cdots (2)$$

The user can evaluate that the larger slope a of the approximate straight line, which is a main evaluation item, is closer to changes in the brain activity of young people and therefore is preferable. When it is difficult to evaluate the main evaluation item, the user can evaluate that the larger intercept b of the approximate straight line as a secondary evaluation item is closer to changes in the brain activity of young people and therefore is preferable. Furthermore, the user can evaluate the reliability of the main evaluation item using the determination coefficient $R^2$. The user can evaluate as the determination coefficient $R^2$ is closer to 1, the effectiveness of the main evaluation item is higher.

When the calculated determination coefficient $R^2$ is less than a preset threshold, the controller 21 determines the presence or absence of the plateau region T (see FIG. 6) as a stagnant region in which the brain activity amount for each work performance is within a predetermined range.

Specifically, as shown in FIG. 6, it is assumed that five plots of the brain activity amount with respect to the work performance are attached. In this case, when the slope of the approximate straight line calculated using a plot of the brain activity amount with respect to the lowest work performance (a rightmost plot in the graph) and a plot of the brain activity with respect to the second lowest work performance (a second rightmost plot in the graph) is within a predetermined range, the controller 21 determines that a region between the above two plots (a portion indicated by a broken line) is the plateau region T as the stagnant region.

Evaluation of Suitability of Training Using Brain Workload Index

The evaluation of suitability of training using the brain workload index according to one embodiment of the present invention is now described with reference to FIGS. 7 to 12. In this embodiment, the brain workload index calculated by the data processing device is used to evaluate suitability of training. In order to simplify the following description, the number of plots of the brain activity amount with respect to the work performance shown in FIGS. 7 to 12 is two.

As described above, as the brain workload index, the young model is preferable to the elderly model. Therefore, when the brain workload index calculated after the training changes to the young model as compared with the brain workload index calculated before the training (when the slope, which is the amount of change in brain activity with respect to the amount of change in work performance, increases), it can be evaluated that the training is suitable for the subject P. When the brain workload index calculated after the training changes to the elderly model as compared with the brain workload index calculated before the training (when the slope, which is the amount of change in brain activity with respect to the amount of change in work performance, decreases), it can be evaluated that the training is not suitable for the subject P.

Before and after the training, the subject P is caused to perform the plurality of tasks with different difficulty levels. The data processing device 2 acquires brain activity measurement data before the training, brain activity measurement data after the training, and work performance for each task from the brain activity measurement device 1. As shown in FIGS. 7 to 12, the controller 21 calculates a first approximate straight line (before the training) 41 for plotting the brain activity amount with respect to the work performance for each of the plurality of tasks with different difficulty levels before the training. The controller 21 also calculates a second approximate straight line (after the training) 42 for plotting the brain activity amount with respect to the work performance for each of the plurality of tasks with different difficulty levels after the training. In FIGS. 7 to 12, plots of the brain activity amount with respect to the work performance are shown by black circles.

Then, the slope of the calculated first approximate straight line 41 is compared with the slope of the calculated second approximate straight line 42. At this time, as shown in FIGS. 7 to 9, when the slope of the second approximate straight line 42 is greater than the slope of the first approximate straight line 41, the brain workload index after the training can be said to have changed to the young model as compared with the brain workload index before the training. In this case, the user can evaluate that the training is suitable for the subject P. In FIGS. 7 to 12, an actual measured value refers to the value of the feature amount of the brain activity at a work performance value acquired after the training. Furthermore, a predicted value refers to the value of the feature amount of the brain activity at a work performance value (indicated by a white circle) acquired after the training on the calculated first approximate straight line.

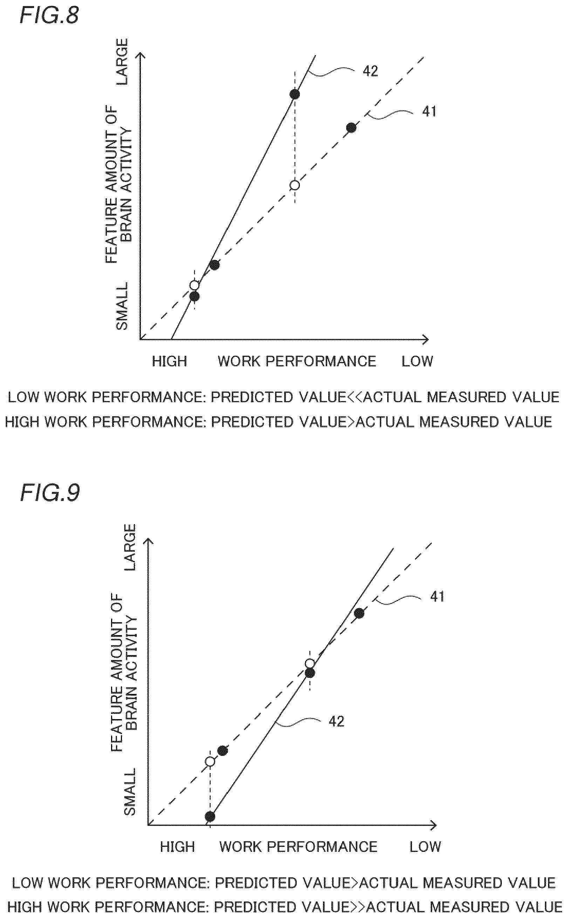

As a result of comparing the first approximate straight line 41 with the second approximate straight line 42 in FIG. 7, the work performance and the brain activity amount have increased overall after the training. Furthermore, as a result of comparing the first approximate straight line 41 with the second approximate straight line 42 in FIG. 8, after the training, on the higher work performance side, the work performance has increased while the brain activity amount has decreased, and on the lower work performance side, the work performance and the brain activity amount have increased. Moreover, as a result of comparing the first approximate straight line 41 with the second approximate straight line 42 in FIG. 9, overall, the work performance has increased while the brain activity amount has decreased after the training. In other words, the amount of change in brain activity with respect to the amount of change in work performance (the slope of the approximate straight line) is meaningful and important. In all of FIGS. 7 to 9, the amount of change in brain activity with respect to the amount of change in work performance (the slope of the approximate straight line) has increased after the training. In the case shown in FIG. 9, it is possible that the subject became accustomed to the task through repeated training.

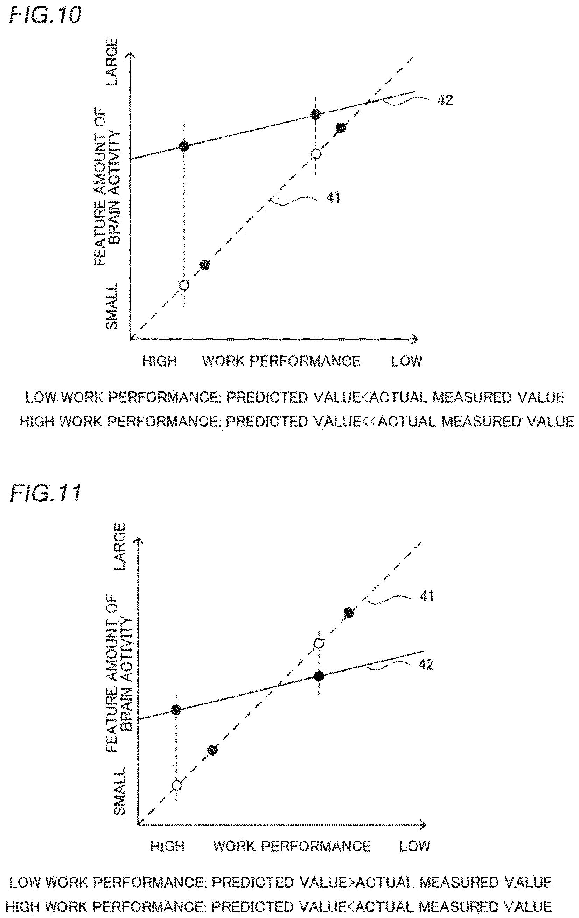

On the other hand, as shown in FIGS. 10 to 12, when the slope of the second approximate straight line 42 is smaller than the slope of the first approximate straight line 41, the brain workload index after the training can be said to have changed to the elderly model as compared with the brain workload index before the training. In this case, the user can evaluate that the training is not suitable for the subject P.

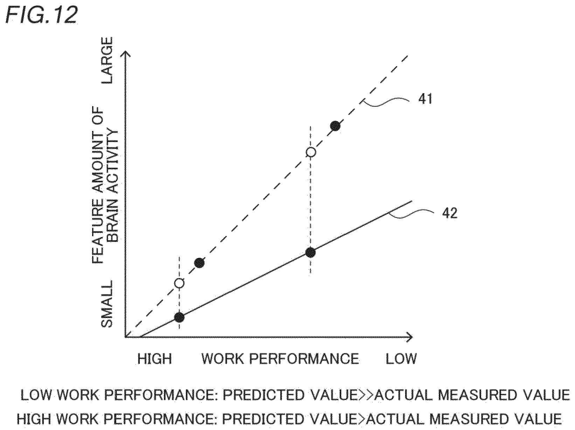

As a result of comparing the first approximate straight line 41 with the second approximate straight line 42 in FIG. 10, the work performance and the brain activity amount have increased overall after the training. Furthermore, as a result of comparing the first approximate straight line 41 with the second approximate straight line 42 in FIG. 11, after the training, on the higher work performance side, the work performance and the brain activity amount have increased, and on the lower work performance side, the work performance has increased while the brain activity amount has decreased. Moreover, as a result of comparing the first approximate straight line 41 with the second approximate straight line 42 in FIG. 12, overall, the work performance has increased while the brain activity amount has decreased after the training. In all of FIGS. 10 to 12, the amount of change in brain activity with respect to the amount of change in work performance (the slope of the approximate straight line) has decreased after the training.

The brain workload index before and after the training is calculated for the same subject P such that it is possible to objectively show whether the training is suitable or not. When the brain workload index calculated after the training is larger than the brain workload index calculated before the training, it can be evaluated that the training is suitable for the subject P. When the brain workload index calculated after the training is smaller than the brain workload index calculated before the training, it can be evaluated that the training is not suitable for the subject P.

Brain Workload Index Calculation Process

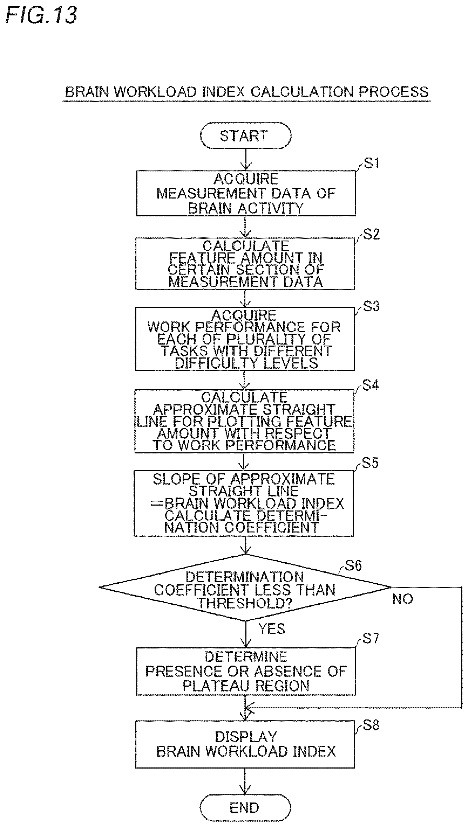

A brain workload index calculation process according to this embodiment is now described with reference to FIG. 13. The brain workload index calculation process described below is executed by the controller 21 including a CPU or the like as hardware. The controller 21 acquires brain activity measurement data and work performance before and after predetermined training, and calculates an approximate straight line before and after the predetermined training.

In step S1, the controller 21 acquires, from the brain activity measurement device 1, measurement data of the brain activity of a subject P during execution of a plurality of tasks with different difficulty levels. Then, the process advances to step S2.

In step S2, the controller 21 calculates a feature amount in a certain section of the acquired brain activity measurement data. Then, the process advances to step S3.

In step S3, the controller 21 acquires work performance for each of the plurality of tasks with different difficulty levels input through the input 23. Then, the process advances to step S4.

In step S4, the controller 21 calculates an approximate straight line for plotting the calculated brain activity feature amount with respect to the acquired work performance. Then, the process advances to step S5.

In step S5, the controller 21 uses at least the slope of the calculated approximate straight line as the brain workload index. Furthermore, the controller 21 calculates a determination coefficient $R^2$ indicating the ac-

curacy of the approximate straight line. Then, the process advances to step S6.

In step S6, the controller 21 determines whether or not the calculated determination coefficient $R^2$ is less than a preset threshold. When the calculated determination coefficient $R^2$ is less than the threshold (Yes in step S6), the process advances to step S7, and when the calculated determination coefficient $R^2$ is equal to or greater than the threshold (No in step S6), the process advances to step S8.

In step S7, the controller 21 determines the presence or absence of the plateau region T in the feature amount of the brain activity. Then, the process advances to step S8.

In step S8, the controller 21 causes the display 24 to display the brain workload index. Then, the process is terminated.

Advantages of Brain Workload Index Calculation System According to This Embodiment

In the brain workload index calculation system 100 according to this embodiment, the following advantages are obtained.

In this embodiment, as described above, the brain workload index calculation system 100 includes the brain activity measurement device 1, the controller 21 configured or programmed to calculate the brain workload index based on the degree of change in the measurement data of the brain activity of the subject P during execution of the plurality of tasks with different difficulty levels with respect to the work performance of the subject P for each of the plurality of tasks with different difficulty levels, acquired from the brain activity measurement device 1, and the display 24 configured to display the calculated brain workload index. The "work performance" is a score representing an evaluation of a result for a task performed by the subject P during brain activity measurement. The degree of change in the measurement data of the brain activity of the subject P with respect to the work performance of the subject P is acquired as the brain workload index such that it can be evaluated that the larger degree of change acquired is closer to changes in the brain activity of young people. Therefore, it is possible to present an index showing the brain workload that can be used as objective data indicating the state of the brain when a worker performs work.

In this embodiment, as described above, the controller 21 is configured or programmed to calculate, as the brain workload index, the amount of change in brain activity in the measurement data with respect to the amount of change in order of work performance for each of the plurality of tasks with different difficulty levels. The amount of change in the order of work performance is used rather than the amount of change in order of difficulty for the work performance acquired for the plurality of tasks with different difficulty levels such that the amount of change in brain activity with respect to the amount of change in work performance can be quantified and acquired, and thus it is possible to present an index showing the brain workload that can be used as objective and clear data indicating the state of the brain.

In this embodiment, as described above, the controller 21 is configured or programmed to acquire the work performance obtained by converting the evaluations of the results for the plurality of tasks with different difficulty levels into scores. Accordingly, the work performance can be quantified and acquired. The amount of change in work performance varies depending on a work performance value, and thus it is possible to appropriately and precisely calculate the amount of change in brain activity with respect to the amount of change in work performance as the brain workload index.

In this embodiment, as described above, the controller 21 is configured or programmed to calculate the approximate straight line for the plot points plotted on the graph as the brain activity amount based on the amount of change in brain activity with respect to the work performance for each of the plurality of tasks with different difficulty levels, and use the slope of the calculated approximate straight line as the brain workload index. Accordingly, the amount of change in brain activity with respect to the amount of change in work performance can be easily quantified and acquired, and thus it is possible to present an index showing the brain workload that can be used as more objective data indicating the state of the brain when the worker performs work.

In this embodiment, as described above, the display 24 is configured to display the graph representing the approximate straight line along with the brain workload index. Accordingly, the user or subject P can be caused to visually and intuitively recognize the brain workload index.

In this embodiment, as described above, the display 24 is configured to display the graph in which the vertical axis represents the brain activity amount, the horizontal axis represents the work performance, and the work performance decreases as the distance from the origin increases. The brain activity tends to increase as the work performance decreases (as the difficulty level of the task increases), and thus the approximate straight line tends to slope upward to the right in the graph configured as described above. Accordingly, it is possible to display the approximate straight line that slopes upward to the right in the graph, and thus the user or subject P can be caused to more visually and intuitively recognize the brain workload index.

In this embodiment, as described above, the display 24 is configured or programmed to display the graph representing, as the approximate straight line, the first approximate straight line 41 for the plot points plotted on the graph as the brain activity amount with respect to the work performance for each of the plurality of tasks with different difficulty levels before the predetermined training, and the second approximate straight line 42 for the plot points plotted on the graph as the brain activity amount with respect to the work performance for each of the plurality of tasks with different difficulty levels after the predetermined training. Accordingly, the user or subject P can be caused to visually and intuitively recognize the brain workload index before and after the predetermined training, and thus it is possible to easily recognize the evaluation of the suitability of the training.

In this embodiment, as described above, the controller 21 is configured or programmed to determine the presence or absence of the plateau region T as the stagnant region in which the brain activity amount for each work performance is within the predetermined range. Accordingly, the user can easily recognize the presence or absence of the plateau region T that serves as an indicator that an increase in the brain activity amount has stagnated.

In this embodiment, as described above, the brain activity measurement device 1 is configured to measure the brain activity of the prefrontal cortex in the head of the subject P. Accordingly, it is possible to present an index showing the workload of regions of the brain related to memory, cognition, motivation, judgment, etc.

In this embodiment, as described above, the brain activity measurement device 1 is configured to optically measure the brain activity of the subject P using near-infrared spectroscopy. Accordingly, large-scale equipment is not required as compared with magnetic resonance imaging (MRI), for example, and thus changes in the brain activity of the subject P can be easily measured.

Advantages of Brain Workload Index Calculation Method According to This Embodiment

As described above, the brain workload index calculation method according to this embodiment includes a step of calculating the brain workload index based on the degree of change in the measurement data of the brain activity of the subject P during execution of the plurality of tasks with different difficulty levels with respect to the work performance of the subject P for each of the plurality of tasks with different difficulty levels. Similarly to the brain workload index calculation system described above, the degree of change in the measurement data of the brain activity of the subject P with respect to the work performance of the subject P is acquired as the brain workload index such that it can be evaluated that the larger degree of change acquired is closer to changes in the brain activity of young people. Therefore, it is possible to present an index showing the brain workload that can be used as objective data indicating the state of the brain when the worker performs work.

In the brain workload index calculation method according to this embodiment, as described above, a step of acquiring the measurement data includes a step of causing the subject P to perform the plurality of tasks with different difficulty levels in order from a task with the lowest difficulty level to a task with the highest difficulty level and acquiring the measurement data of the brain activity of the subject P during execution of the plurality of tasks. It is possible to reduce or prevent the possibility that the subject P gives up performing the task midway through by changing the difficulty level of the task from a low difficulty level to a high difficulty level in stages.

In the brain workload index calculation method according to this embodiment, as described above, the step of acquiring the measurement data includes a step of acquiring the measurement data of the brain activity of the subject P before the predetermined training and a step of acquiring the measurement data of the brain activity of the subject P after the predetermined training, and a step of acquiring the work performance includes a step of acquiring the work performance of the subject P for each of the plurality of tasks before the predetermined training, and a step of acquiring the work performance of the subject P for each of the plurality of tasks after the predetermined training. Accordingly, the brain workload index before and after the predetermined training can be easily calculated.

In the brain workload index calculation method according to this embodiment, as described above, the step of calculating the brain workload index includes a step of calculating the approximate straight line for the plot points plotted on the graph as the brain activity amount based on the measurement data with respect to the work performance for each of the plurality of tasks before the predetermined training and using the slope of the calculated approximate straight line as the brain workload index before the predetermined training, and a step of calculating the approximate straight line for the plot points plotted on the graph as the brain activity amount with respect to the work performance for each of the plurality of tasks after the predetermined training and using the slope of the calculated approximate straight line as the brain workload index after the predetermined training. Accordingly, the suitability of the training can be evaluated based on the slope of the calculated approximate straight line.

Modified Examples

The embodiment disclosed this time must be considered as illustrative in all points and not restrictive. The scope of the present invention is not shown by the above description of the embodiment but by the scope of claims for patent, and all modifications (modified examples) within the meaning and scope equivalent to the scope of claims for patent are further included.

For example, while the example in which the brain workload index is used to evaluate the suitability of the training has been shown in the aforementioned embodiment, the present invention is not limited to this. For example, the brain workload index may be used for product evaluation in product development. In the aforementioned embodiment, the brain workload index is calculated before and after the training, but in a modified example, the brain workload index is calculated when a different product is used.

As described above, as the brain workload index, the young model is preferable to the elderly model. Assume that approximate straight lines are acquired for plotting the brain activity amount with respect to the work performance for each of the plurality of tasks with different difficulty levels when a product A and a product B are used. When the approximate straight line for the product A is compared with the approximate straight line for the product B, the product with a larger brain activity amount at $+\infty$ in a case of estimating a difficulty level of $+\infty$ and a smaller brain activity amount at $-\infty$ in a case of estimating a difficulty level of $-\infty$ can be estimated as a product that exhibits brain activity closer to that of the young model.

For example, assume that the approximate straight line for the product A has a larger slope than the approximate straight line for the product B, which is the amount of change in brain activity with respect to the amount of change in work performance when the approximate straight line for the product A is compared with the approximate straight line for the product B. In this case, the product A can be evaluated as a product that exhibits brain activity closer to that of the young model. Moreover, the product A can be evaluated as a product that can respond to the brain activity of the subject P according to the difficulty level better than the product B.

Whether it is preferable for a brain workload index of a product to more closely approximate the young model depends on the purpose and use of the product. In an example in which the brain workload index of the product A approximates the young model than the brain workload index of the product B, the product A is conceivably a product that is easier for the user to apply (easier to use) than the product B when the product A and the product B are products, the purposes of which are to enable efficient work, for example. On the other hand, the product B is conceivably a product that is easier for the user to apply (easier to use) than the product A when the product A and the product B are products, the purposes of which are to maintain a state of mental and physical rest.

Product Evaluation Using Brain Workload Index

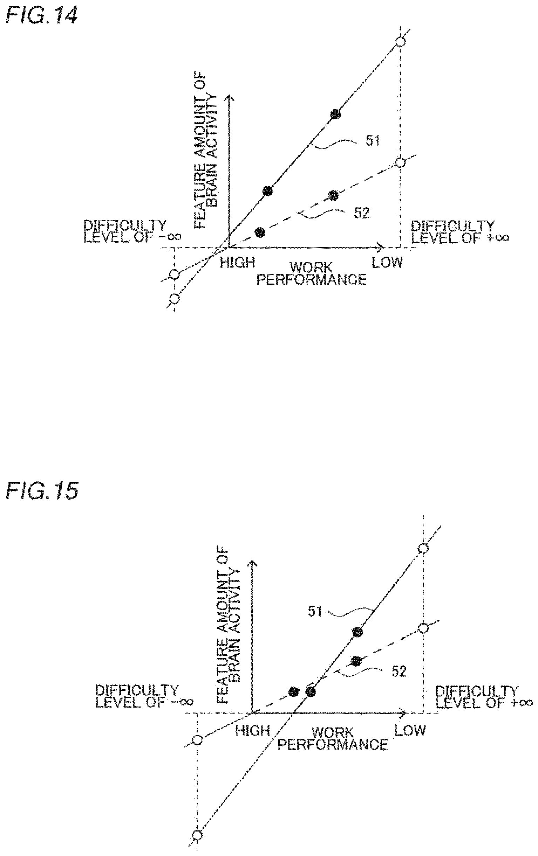

Product evaluation using the brain workload index is now described with reference to FIGS. 14 to 16. Similarly to the embodiment described above, the subject P is caused to perform the plurality of tasks with different difficulty levels when using the product A. The data processing device 2 acquires measurement data of brain activity and work performance for each task. The controller 21 calculates a first approximate straight line 51 for plotting a brain activity amount with respect to the work performance for each of the plurality of tasks with different difficulty levels for the product A. Furthermore, the subject P is caused to perform the plurality of tasks with different difficulty levels when using the product B. The data processing device 2 acquires measurement data of brain activity and work performance for each task. The controller 21 calculates a second approximate straight line 52 for plotting the brain activity amount with respect to the work performance for each of the plurality of tasks with different difficulty levels for the product B. Then, the slope of the calculated first approximate straight line 51 is compared with the slope of the calculated second approximate straight line 52. At this time, as shown in FIGS. 14 to 16, when the slope of the first approximate straight line 51 for the product A is larger than the slope of the second approximate straight line 52 for the product B, it can be estimated that the brain workload index of the product A more closely approximates the young model than the brain workload index of the product B. In FIGS. 14 to 16, plot points of the brain activity amount with respect to the work performance are shown by black circles, and plot points of the brain activity amount with respect to the work performance when estimating the $+\infty$ difficulty level and $-\infty$ difficulty level are shown by white circles.

The number of products to be compared in product evaluation may be two, or may be three or more. Furthermore, the plurality of tasks with different difficulty levels may be tasks using the product itself or may be tasks not using the product itself, as long as the tasks are performed when the product is used.

Examples of using the brain workload index are not limited to evaluation of suitability of training and product evaluation in product development. Another example of using the brain workload index includes calculating the brain workload indexes of different subjects P and evaluating which subject P's brain workload index more closely approximates the young model.

While the example in which the controller 21 determines the presence or absence of the plateau region T when the calculated determination coefficient $R^2$ is less than the preset threshold has been shown in the aforementioned embodiment, the present invention is not limited to this. When determining that the plateau region T is present, the controller 21 may calculate an approximate straight line excluding plot points included in the plateau region T (plot points of the brain activity amount with respect to the work performance). The slope of an approximate straight line calculated including the plot points included in the plateau region T is smaller than the slope of the approximate straight line calculated excluding the plot points included in the plateau region T, and thus it may not be appropriate as the brain workload index. The approximate straight line excluding the plot points included in the plateau region T is calculated such that it may be possible to calculate an appropriate brain workload index.

While the example in which the controller 21 determines the presence or absence of the plateau region T when the calculated determination coefficient $R^2$

is less than the preset threshold has been shown in the aforementioned embodiment, the present invention is not limited to this. For example, when the subject P is caused to perform a plurality of tasks with a predetermined number or more of different difficulty levels and the calculated determination coefficient $R^2$ is less than the preset threshold, the controller 21 may determine the presence or absence of the plateau region T. For example, the subject P may be caused to perform a plurality of tasks with five or more different difficulty levels as the predetermined number. Alternatively, the controller 21 may determine the presence or absence of the plateau region T even when the calculated determination coefficient $R^2$ is equal to or greater than the preset threshold.

The user may select at least two of a plurality of plot points of the brain activity amount with respect to the work performance for each of the plurality of tasks with different difficulty levels shown in the graph, and the controller 21 may calculate an approximate straight line based on the plot points selected by the user. Thus, it is possible to appropriately acquire the brain workload index in a region in which the user desires to acquire the brain workload index.

While the example in which the approximate straight line for plotting the brain activity amount with respect to the work performance for each of the plurality of tasks with different difficulty levels is calculated has been shown in the aforementioned embodiment, the present invention is not limited to this. For example, an approximate curve for plotting the brain activity amount with respect to the work performance for each of the plurality of tasks with different difficulty levels may be calculated.

While the example in which changes in the brain activity of the subject P are measured using a NIRS device has been shown in the aforementioned embodiment, the present invention is not limited to this. Changes in the brain activity of the subject P may be measured using an MRI device (magnetic resonance imaging device) or an electroencephalograph, for example.

While the example in which the brain activity of the prefrontal cortex in the head of the subject P is measured has been shown in the aforementioned embodiment, the present invention is not limited to this. The measurement site of the subject P can be set as appropriate.

While the example in which the work performance is input by the user has been shown in the aforementioned embodiment, the present invention is not limited to this. For example, an input 23 may be provided in a processing device provided separately from the data processing device 2. In this case, the processing device including the input 23 is communicably connected to the data processing device 2 and transmits the input work performance to the data processing device 2. Alternatively, the data processing device 2 may acquire, as the work performance, input data input by the subject P using a keyboard of a PC, a tablet terminal, a touch panel of a smartphone, etc. Alternatively, input data by the user regarding numerical values related to work performance may be stored in a database (not shown), and the data processing device 2 may acquire, as the work performance, the input data from the database.

Aspects

First Aspect

It will be appreciated by those skilled in the art that the exemplary embodiments described above are specific examples of the following first aspect.

(Item 1)

A brain workload index calculation system comprising:

a brain activity measurement device configured to measure brain activity of a subject;
a data processor configured or programmed to calculate a brain workload index based on a degree of change in measurement data of the brain activity of the subject during execution of a plurality of tasks with different difficulty levels with respect to work performance of the subject for each of the plurality of tasks with different difficulty levels, the measurement data being acquired from the brain activity measurement device; and
a display configured to display the brain workload index calculated by the data processor.

(Item 2)

The brain workload index calculation system according to item 1, wherein the data processor is configured or programmed to calculate, as the brain workload index, an amount of change in brain activity in the measurement data with respect to an amount of change in order of the work performance for each of the plurality of tasks with different difficulty levels.

(Item 3)

The brain workload index calculation system according to item 2, wherein the data processor is configured or programmed to acquire the work performance obtained by converting evaluations of results for the plurality of tasks with different difficulty levels into scores.

(Item 4)

The brain workload index calculation system according to item 2 or 3, wherein the data processor is configured or programmed to calculate an approximate straight line for plot points plotted on a graph as a brain activity amount based on the amount of change in brain activity with respect to the work performance for each of the plurality of tasks with different difficulty levels, and use a slope of a calculated approximate straight line as the brain workload index.

(Item 5)

The brain workload index calculation system according to item 4, wherein the display is configured to display the graph representing the approximate straight line along with the brain workload index.

(Item 6)

The brain workload index calculation system according to item 5, wherein the display is configured to display the graph in which a vertical axis represents a brain activity amount, a horizontal axis represents work performance, and work performance decreases as a distance from an origin increases.

(Item 7)

The brain workload index calculation system according to item 5 or 6, wherein the display is configured to display the graph representing, as the approximate straight line, a first approximate straight line for plot points plotted on the graph as the brain activity amount with respect to the work performance for each of the plurality of tasks with different difficulty levels before predetermined training, and a second approximate straight line for plot points plotted on the graph as the brain activity amount with respect to the work performance for each of the plurality of tasks with different difficulty levels after the predetermined training.

(Item 8)

The brain workload index calculation system according to any one of items 4 to 7, wherein the data processor is configured or programmed to determine a presence or absence of a plateau region as a stagnant region in which the brain activity amount for each work performance is within a predetermined range.

(Item 9)

The brain workload index calculation system according to any one of items 1 to 8, wherein the brain activity measurement device is configured to measure brain activity of a prefrontal cortex in a head of the subject.

(Item 10)

The brain workload index calculation system according to any one of items 1 to 9, wherein the brain activity measurement device is configured to optically measure the brain activity of the subject using near-infrared spectroscopy.

(Item 11)

A brain workload index calculation method comprising:

acquiring measurement data of brain activity of a subject during execution of a plurality of tasks with different difficulty levels;
acquiring work performance of the subject for each of the plurality of tasks with different difficulty levels;
calculating a brain workload index based on a degree of change in acquired measurement data of the brain activity of the subject with respect to acquired work performance of the subject; and

displaying a calculated brain workload index.

(Item 12)

The brain workload index calculation method according to item 11, wherein the acquiring the measurement data includes causing the subject to perform the plurality of tasks with different difficulty levels in order from a task with a lowest difficulty level to a task with a highest difficulty level and acquiring the measurement data of the brain activity of the subject during execution of the plurality of tasks.

(Item 13)

The brain workload index calculation method according to item 11 or 12, wherein
the acquiring the measurement data includes acquiring the measurement data of the brain activity of the subject before predetermined training, and acquiring the measurement data of the brain activity of the subject after the predetermined training; and
the acquiring the work performance includes acquiring the work performance of the subject for each of the plurality of tasks before the predetermined training, and acquiring the work performance of the subject for each of the plurality of tasks after the predetermined training.

(Item 14)

The brain workload index calculation method according to item 13, wherein the calculating the brain workload index includes calculating an approximate straight line for plot points plotted on a graph as a brain activity amount based on the measurement data with respect to the work performance for each of the plurality of tasks before the predetermined training and using a slope of a calculated approximate straight line as the brain workload index before the predetermined training, and calculating an approximate straight line for plot points plotted on the graph as the brain activity amount with respect to the work performance for each of the plurality of tasks after the predetermined training and using a slope of a calculated approximate straight line as the brain workload index after the predetermined training.

Description of Reference Numerals

1: brain activity measurement device
2: data processing device
21: controller
24: display
41: first approximate straight line (before training)
42: second approximate straight line (after training)
100: brain workload index calculation system
P: subject
T: plateau region

**Claims**

1. A brain workload index calculation system comprising:

a brain activity measurement device configured to measure brain activity of a subject;
a data processor configured or programmed to calculate a brain workload index based on a degree of change in measurement data of the brain activity of the subject during execution of a plurality of tasks with different difficulty levels with respect to work performance of the subject for each of the plurality of tasks with different difficulty levels, the measurement data being acquired from the brain activity measurement device; and
a display configured to display the brain workload index calculated by the data processor.

2. The brain workload index calculation system according to claim 1, wherein the data processor is configured or programmed to calculate, as the brain workload index, an amount of change in brain activity in the measurement data with respect to an amount of change in order of the work performance for each of the plurality of tasks with different difficulty levels.

3. The brain workload index calculation system according to claim 2, wherein the data processor is configured or programmed to acquire the work performance obtained by converting evaluations of results for the plurality of tasks with different difficulty levels into scores.

4. The brain workload index calculation system according to claim 2, wherein the data processor is configured or programmed to calculate an approximate straight line for plot points plotted on a graph as a brain activity amount based on the amount of change in brain activity with respect to the work performance for each of the plurality of tasks with different difficulty levels, and use a slope of a calculated approximate straight line as the brain workload index.

5. The brain workload index calculation system according to claim 4, wherein the display is configured to display the graph representing the approximate straight line along with the brain workload index.

6. The brain workload index calculation system according to claim 5, wherein the display is configured to display the graph in which a vertical axis represents a brain activity amount, a horizontal axis represents work performance, and work performance decreases as a distance from an origin increases.

7. The brain workload index calculation system according to claim 5, wherein the display is configured to display the graph representing, as the approximate straight line, a first approximate straight line for plot points plotted on the graph as the brain activity amount with respect to the work performance for each of the plurality of tasks with different difficulty levels before predetermined training, and a second approximate straight line for plot points plotted on the graph as the brain activity amount with respect to the work performance for each of the plurality of tasks with different difficulty levels after the predetermined training.

8. The brain workload index calculation system according to claim 4, wherein the data processor is configured or programmed to determine a presence or absence of a plateau region as a stagnant region in which the brain activity amount for each work performance is within a predetermined range.

9. The brain workload index calculation system according to claim 1, wherein the brain activity measurement device is configured to measure brain activity of a prefrontal cortex in a head of the subject.

10. The brain workload index calculation system according to claim 1, wherein the brain activity measurement device is configured to optically measure the brain activity of the subject using near-infrared spectroscopy.

11. A brain workload index calculation method comprising:

acquiring measurement data of brain activity of a subject during execution of a plurality of tasks with different difficulty levels;
acquiring work performance of the subject for each of the plurality of tasks with different difficulty levels;

calculating a brain workload index based on a degree of change in acquired measurement data of the brain activity of the subject with respect to acquired work performance of the subject; and
displaying a calculated brain workload index.

12. The brain workload index calculation method according to claim 11, wherein the acquiring the measurement data includes causing the subject to perform the plurality of tasks with different difficulty levels in order from a task with a lowest difficulty level to a task with a highest difficulty level and acquiring the measurement data of the brain activity of the subject during execution of the plurality of tasks.

13. The brain workload index calculation method according to claim 11, wherein

the acquiring the measurement data includes acquiring the measurement data of the brain activity of the subject before predetermined training, and acquiring the measurement data of the brain activity of the subject after the predetermined training; and
the acquiring the work performance includes acquiring the work performance of the subject for each of the plurality of tasks before the predetermined training, and acquiring the work performance of the subject for each of the plurality of tasks after the predetermined training.

14. The brain workload index calculation method according to claim 13, wherein the calculating the brain workload index includes calculating an approximate straight line for plot points plotted on a graph as a brain activity amount based on the measurement data with respect to the work performance for each of the plurality of tasks before the predetermined training and using a slope of a calculated approximate straight line as the brain workload index before the predetermined training, and calculating an approximate straight line for plot points plotted on the graph as the brain activity amount with respect to the work performance for each of the plurality of tasks after the predetermined training and using a slope of a calculated approximate straight line as the brain workload index after the predetermined training.

## FIG.1

## FIG.2

*FIG.3*

SUBJECT INFORMATION ⌐30

MEASUREMENT INFORMATION ⌐31

CURRENT WORKLOAD INDEX ⌐32

CURRENT BEHAVIORAL DATA ⌐33

WORKLOAD INDEX HISTORY ⌐34
RIGHT BRAIN ▬
LEFT BRAIN ▭
MONTH/DAY (NUMBER OF TIMES)

BEHAVIORAL DATA HISTORY ⌐35
MONTH/DAY (NUMBER OF TIMES /DIFFICULTY LEVEL)

FIG.4

FIG.5

FIG.6

FIG.7

LOW WORK PERFORMANCE: PREDICTED VALUE≪ACTUAL MEASURED VALUE
HIGH WORK PERFORMANCE: PREDICTED VALUE＜ACTUAL MEASURED VALUE

## FIG.8

LOW WORK PERFORMANCE: PREDICTED VALUE<<ACTUAL MEASURED VALUE
HIGH WORK PERFORMANCE: PREDICTED VALUE>ACTUAL MEASURED VALUE

## FIG.9

LOW WORK PERFORMANCE: PREDICTED VALUE>ACTUAL MEASURED VALUE
HIGH WORK PERFORMANCE: PREDICTED VALUE>>ACTUAL MEASURED VALUE

*FIG.10*

LOW WORK PERFORMANCE: PREDICTED VALUE<ACTUAL MEASURED VALUE

HIGH WORK PERFORMANCE: PREDICTED VALUE<<ACTUAL MEASURED VALUE

*FIG.11*

LOW WORK PERFORMANCE: PREDICTED VALUE>ACTUAL MEASURED VALUE

HIGH WORK PERFORMANCE: PREDICTED VALUE<ACTUAL MEASURED VALUE

FIG.12

LOW WORK PERFORMANCE: PREDICTED VALUE>>ACTUAL MEASURED VALUE

HIGH WORK PERFORMANCE: PREDICTED VALUE>ACTUAL MEASURED VALUE

# FIG.13

**BRAIN WORKLOAD INDEX CALCULATION PROCESS**

```
        ( START )
           │
           ▼
  ┌──────────────────┐  S1
  │     ACQUIRE       │
  │ MEASUREMENT DATA OF│
  │  BRAIN ACTIVITY   │
  └──────────────────┘
           │
           ▼
  ┌──────────────────┐  S2
  │    CALCULATE      │
  │ FEATURE AMOUNT IN │
  │ CERTAIN SECTION OF│
  │  MEASUREMENT DATA │
  └──────────────────┘
           │
           ▼
  ┌──────────────────┐  S3
  │     ACQUIRE       │
  │ WORK PERFORMANCE FOR│
  │ EACH OF PLURALITY OF│
  │ TASKS WITH DIFFERENT│
  │  DIFFICULTY LEVELS │
  └──────────────────┘
           │
           ▼
  ┌──────────────────┐  S4
  │    CALCULATE      │
  │ APPROXIMATE STRAIGHT│
  │LINE FOR PLOTTING FEATURE│
  │  AMOUNT WITH RESPECT│
  │  TO WORK PERFORMANCE│
  └──────────────────┘
           │
           ▼
  ┌──────────────────┐  S5
  │ SLOPE OF APPROXIMATE│
  │   STRAIGHT LINE   │
  │ =BRAIN WORKLOAD INDEX│
  │  CALCULATE DETERMI-│
  │  NATION COEFFICIENT│
  └──────────────────┘
           │
           ▼
       ╱────────╲  S6
      ╱ DETERMINATION╲
     ╱ COEFFICIENT LESS THAN ╲───── NO ──┐
      ╲   THRESHOLD?  ╱                    │
       ╲────────╱                          │
           │ YES                           │
           ▼                               │
  ┌──────────────────┐  S7                 │
  │    DETERMINE      │                    │
  │ PRESENCE OR ABSENCE OF│                │
  │  PLATEAU REGION   │                    │
  └──────────────────┘                     │
           │ ◄──────────────────────────────┘
           ▼
  ┌──────────────────┐  S8
  │     DISPLAY       │
  │ BRAIN WORKLOAD INDEX│
  └──────────────────┘
           │
           ▼
        (  END  )
```

22

FIG.14

FIG.15

*FIG.16*

# INTERNATIONAL SEARCH REPORT

| International application No. |
| --- |
| **PCT/JP2022/020381** |

## A. CLASSIFICATION OF SUBJECT MATTER

*G06Q 50/10*(2012.01)i; *A61B 10/00*(2006.01)i
FI:  G06Q50/10; A61B10/00 E

According to International Patent Classification (IPC) or to both national classification and IPC

## B. FIELDS SEARCHED

Minimum documentation searched (classification system followed by classification symbols)

G06Q50/10; A61B10/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
| --- | --- | --- |
| A | JP 2020-130630 A (SHIMADZU CORP) 31 August 2020 (2020-08-31) entire text, all drawings | 1-14 |
| A | JP 2018-158042 A (UNIV TOHOKU) 11 October 2018 (2018-10-11) entire text, all drawings | 1-14 |
| A | JP 2018-140162 A (PANASONIC IP MAN CORP) 13 September 2018 (2018-09-13) entire text, all drawings | 1-14 |

☐ Further documents are listed in the continuation of Box C.          ☑ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| **22 July 2022** | **09 August 2022** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/020381**

| Patent document cited in search report | | | Publication date (day/month/year) | Patent family member(s) | | | Publication date (day/month/year) |
|---|---|---|---|---|---|---|---|
| JP | 2020-130630 | A | 31 August 2020 | US | 2020/0261015 | A1 | |
| | | | | entire text, all drawings | | | |
| | | | | KR | 10-2020-0101850 | A | |
| | | | | CN | 111588348 | A | |
| JP | 2018-158042 | A | 11 October 2018 | (Family: none) | | | |
| JP | 2018-140162 | A | 13 September 2018 | US | 2019/0300001 | A1 | |
| | | | | entire text, all drawings | | | |
| | | | | WO | 2018/159276 | A1 | |
| | | | | EP | 3590426 | A1 | |
| | | | | CN | 109890288 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2015229095 A